# FASCICULE DE BREVET EUROPEEN

(11) **EP 3 986 704 B1**
(45) Date de publication et mention de la délivrance du brevet: **07.06.2023**
(21) Numéro de dépôt: 20740062.3
(22) Date de dépôt: 22.05.2020
(51) Int. Cl.: B29C 64/35, B29C 64/357, B33Y 30/00, B33Y 40/20, B29C 64/153, B29C 64/314, B29C 64/386, B01L 1/04, B29C 64/25, B33Y 50/00, C12M 1/12

(54) **INSTALLATION D'IMPRESSION EN TROIS DIMENSIONS D'UN DISPOSITIF MÉDICAL**
INSTALLATION ZUM DREIDIMENSIONALEN DRUCKEN EINER MEDIZINISCHEN VORRICHTUNG
INSTALLATION FOR THE THREE-DIMENSIONAL PRINTING OF A MEDICAL DEVICE

(30) Priorité: 24.06.2019 FR 1906770
(43) Date de publication de la demande: 27.04.2022
(73) Titulaire: Med in Town, 42400 Saint-Chamond (FR)
(72) Inventeur: PRECHEUR, Jérôme, 42400 SAINT-CHAMOND (FR)
(74) Mandataire: Cabinet Laurent & Charras
(86) Numéro de dépôt international: PCT/FR2020/050854
(87) Numéro de publication internationale: WO 2020/260781

(56) Documents cités:
- WO-A1-2018/150231
- CN-A- 106 901 770
- FR-A1- 3 058 338
- US-A1- 2016 010 883
- US-A1- 2019 009 334

## Description

### DOMAINE TECHNIQUE

L'invention se rattache au secteur technique de la fabrication de dispositifs médicaux, c'est-à-dire tout instrument, appareil ou équipement, utilisé pour le diagnostic, la prévention ou le traitement d'une maladie, d'une blessure ou d'un handicap, ou pour la réalisation d'une opération chirurgicale.

L'invention concerne plus particulièrement une installation d'impression en trois dimensions d'un tel dispositif médical, directement sur le lieu où il doit être utilisé, par exemple dans un centre hospitalier.

### ART ANTERIEUR

Dans l'état de la technique, les centres hospitaliers doivent se procurer un nombre considérable de dispositifs médicaux, c'est-à-dire d'instruments, ancillaires, implants d'essai, ou tout autre élément destiné à être utilisé pour le diagnostic, la prévention, le traitement d'une maladie, d'une blessure, ou d'un handicap, ou pour la réalisation d'une opération chirurgicale.

Le nombre considérable de ces dispositifs médicaux rend complexe leur traçage et suivi, de sorte qu'il arrive parfois que l'un ou plusieurs de ces dispositifs médicaux ne soient pas livrés à temps, compromettant alors la pratique médicale.

Par ailleurs, une grande partie de ces dispositifs médicaux est réalisée en métal et nécessite, après utilisation, d'être transportée pour être traitée et remis en état, et livrée de nouveau vers les centres hospitaliers.

Le suivi et la gestion de ces différentes opérations sont donc coûteux et fastidieux, et génèrent un risque pour le patient final.

Par ailleurs, pour un centre hospitalier par exemple, le processus de commande d'un dispositif médical est lourd et complexe et fait intervenir un nombre important d'interlocuteurs et de fournisseurs. La recherche des dispositifs médicaux bien adaptés aux patients en devient donc fastidieuse.

Au surplus, les commandes se font généralement en gros, ce qui implique la gestion d'un stock, avec des produits identifiés et conditionnés de manières différentes en fonction des fournisseurs.

Certains hôpitaux ont tenté de s'équiper avec des imprimante 3D, sans succès, et avec d'importantes difficultés pour appréhender le processus total d'impression jusqu'au produit final emballé.

Il est connu des documents CN106901770 et US2016/010883 des installations d'impression en trois dimensions d'un dispositif médical directement sur le lieu où le dispositif médical doit être utilisé, qui présentent l'inconvénient d'être complexes et coûteuses.

### EXPOSE DE L'INVENTION

L'un des buts de l'invention est donc de remédier aux problèmes précités en fournissant une installation permettant d'éviter les aléas liés aux traitements et à la livraison des dispositifs médicaux.

Un autre objectif de l'invention est de simplifier le traçage et le suivi desdits dispositifs médicaux.

L'invention vise également à diminuer le coût et le temps de commande et de gestion desdits dispositif médicaux, tout en permettant de choisir les dispositifs médicaux les mieux adaptés aux patients.

Selon l'invention, l'installation comprend un container comprenant intérieurement :
- un module de production comprenant une imprimante 3D ;
- une salle blanche, distincte du module de production, comprenant des moyens de lavage et de désinfection du dispositif médical imprimé, et une machine d'emballage du dispositif médical lavé et désinfecté.

De ce qui précède, l'installation permet de fabriquer un dispositif médical, au sein même du lieu où il doit être utilisé, en circuit court, supprimant ainsi les aléas liés aux transports. La fabrication du dispositif médical est assurée en toute transparence, dans des conditions optimales en permettant un usage hospitalier, par exemple dans un bloc opératoire. La salle blanche selon la norme ISO 14644-1 comprend des moyens de maitrise de la propreté de l'air. Ces moyens comprennent un groupe de filtration permettant de maitriser la concentration particulaire et d'éviter toute contamination microbienne à l'intérieur de la salle blanche. Les paramètres tels que la température, l'humidité et la pression relative sont également contrôlés et maintenus à un niveau précis.

Les dispositifs médicaux imprimés sont tous identifiés et emballés d'une manière homogène de sorte que leur traçage et suivi sont améliorés.

Selon une forme de réalisation particulière, l'imprimante 3D est une machine de frittage sélectif de poudre par laser, connue sous l'acronyme « SLS » de l'anglais « Sélective Laser Sintering », et le module de production comprend une station de déballage d'un dispositif médical imprimé, et une station de dépoudrage d'un dispositif médical déballé.

Avantageusement, le module de production comprend également une mélangeuse de poudre, permettant de préparer directement dans le container une poudre de matériaux à imprimer, issue par exemple d'une opération de recyclage.

Le dépoudrage est effectué par toute technique appropriée. Par exemple, la station de dépoudrage comprend un dispositif de dépoudrage par sablage.

Avantageusement, le module de production comprend une station de contrôle et de reprise du dispositif médical.

Selon une forme de réalisation particulière, la salle blanche comprend un dispositif de stérilisation, de préférence de type autoclave.

De préférence, le module de production communique avec la salle blanche par l'intermédiaire d'un sas.

Selon une forme de réalisation particulière, l'imprimante 3D est commandée par une interface homme-machine configurée pour permettre à un utilisateur de sélectionner un dispositif médical à imprimer accessible depuis une base de données.

### DESCRIPTION DES FIGURES

[Fig. 1] la figure 1 est une représentation schématique illustrant, en perspective, l'installation selon l'invention ;
[Fig. 2] la figure 2 est une représentation schématique de l'installation de la figure 1, vue de dessus ;
[Fig. 3] la figure 3 est une représentation schématique de l'installation de la figure 1, vue de face.

### DESCRIPTION DETAILLEE DE L'INVENTION

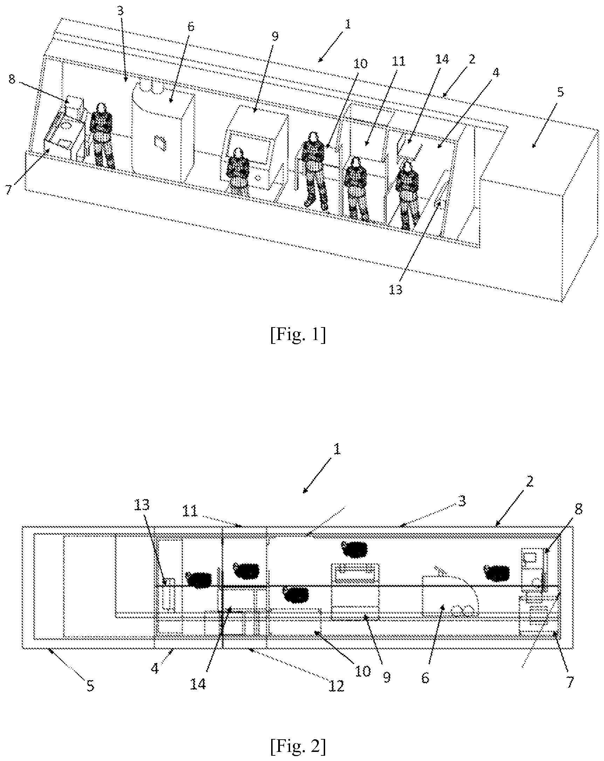

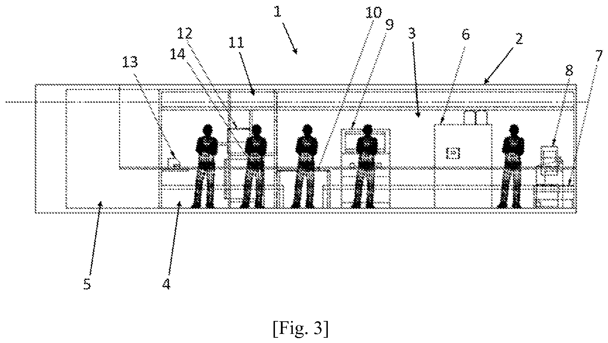

En référence aux figures 1 à 3, l'invention concerne une installation (1) d'impression en trois dimensions d'un dispositif médical, comprenant un container (2) adapté pour être positionné directement sur le lieu où le dispositif médical doit être utilisé, par exemple dans un centre hospitalier.

L'invention permet alors d'imprimer directement sur place des dispositifs médicaux, et d'éviter les aléas du traitement et du transport de ces dispositifs, tout en garantissant un usage hospitalier, par exemple dans un bloc opératoire.

À cet effet, le container (2) comprend intérieurement un module de production (3), une salle blanche (4) distincte du module de production, et de préférence un local technique (5).

Le module de production (3) comprend au moins une imprimante 3D (6), de préférence du type machine de frittage de poudre SLS.

Le module de production (3) peut être configuré pour permettre de travailler avec une poudre recyclée pour alimenter l'imprimante 3D (6). À cet effet, le module de production (3) comprend avantageusement une mélangeuse (7) de poudre, éventuellement recyclée, pour préparer la poudre avant chargement dans l'imprimante 3D (6). La poudre utilisée est recyclable, par exemple en polyamide.

Le module de production (3) comprend ensuite une station de déballage (8) du dispositif médical imprimé en trois dimensions. La station de déballage (8) permet, de déballer le dispositif médical brut imprimé, en vue de son dépoudrage. Le module de production (3) comprend aussi une station de dépoudrage (9) du dispositif médical déballé, comprenant par exemple un dispositif de dépoudrage par sablage.

D'une manière avantageuse, la station de déballage (8) est adjacente à la mélangeuse (7) de sorte que la poudre issue du déballage est directement acheminée dans la mélangeuse (7) en vue sa réutilisation dans l'imprimante 3D (6).

Le module de production (3) comprend avantageusement une station de contrôle et de reprise (10) du dispositif médical dépoudré, par exemple au moyen de matériels conventionnels du type pied à coulisse ou autre et/ou au moyen d'une rétro conception par scanner 3D.

Le module de production (3) communique avec la salle blanche (4) par l'intermédiaire d'un sas (11). La salle blanche (4) comprend des moyens de lavage et de désinfection (12) du dispositif médical dépoudré, notamment un laveur désinfecteur, et une machine d'emballage (13), notamment sous vide, du dispositif médical lavé et désinfecté. La salle blanche (4) comprend des moyens de maitrise de la propreté de l'air, par exemple sous la forme d'un groupe de filtration permettant de maitriser la concentration particulaire et d'éviter toute contamination microbienne à l'intérieur de la salle blanche (4).

La salle blanche (4) comprend également de préférence un dispositif de stérilisation (14), du type autoclave, pour stériliser le dispositif médical emballé. Le dispositif emballé et stérilisé est ensuite éventuellement sur-emballé, par exemple dans une boîte spécifique en carton au niveau d'une station de sur-emballage (non représentée).

L'emballage et le sur-emballage sont standards au lieu où est utilisé le dispositif médical, par exemple au centre hospitalier, et portent les identifications nécessaires pour assurer le traçage et suivi du dispositif médical.

Le local technique (5) du container (2) permet le traitement de l'air du container (2), la production d'eau, d'air comprimé, et la gestion de l'alimentation électrique des différents équipements, ainsi que des éventuelles alarmes.

De préférence, le container (2) comprend des moyens de collecte (non représentés) des dispositifs médicaux imprimés à jeter, notamment non conformes ou déjà utilisés, en vue de leur recyclage.

L'imprimante 3D (6) du module de production (3) est de préférence commandée par une interface homme-machine configurée pour permettre à un utilisateur de sélectionner un dispositif médical à imprimer accessible depuis une base de données.

Par exemple, l'utilisateur peut correspondre à un pharmacien, un responsable de bloc opératoire, un chirurgien, un enseignant ou tout autre personne.

Sur cette interface homme-machine, l'utilisateur peut sélectionner le dispositif médical à imprimer le mieux adapté au patient en effectuant des recherches, par exemple par critères, par fabricants ou encore par historique de fabrication. Les dispositifs médicaux à imprimer peuvent être affichés sous la forme d'un menu déroulant avec une image en trois dimensions de chaque dispositif médical ainsi qu'un descriptif technique. Par exemple, le descriptif technique comporte des caractéristiques techniques et le prix de chaque dispositif médical.

Pour obtenir cet affichage pour l'utilisateur, l'interface homme-machine est connectée à un serveur de fichiers intégrant un module de gestion électronique de documents. Ce module de gestion électronique de documents intègre préférentiellement le descriptif technique et le plan de fabrication en trois dimensions de chaque dispositif médical.

Le plan de fabrication en trois dimension de chaque dispositif médical peut être stocké sous divers formats. De préférence, le plan de fabrication est stocké sous le format STL qui est utilisé dans les logiciels de stéréolithographie et qui a été développé par la société 3D Systems^{®}.

En complément ou en variante, le module de gestion électronique de documents peut accepter les fichiers de type PDF ou vidéo. De plus, le module de gestion électronique de documents peut être configuré pour effectuer des traitements sur les plans de fabrication afin de les normaliser ou d'en obtenir des informations, telles que des informations de dimension, afin de les présenter à l'utilisateur ou de permettre des recherches ciblées en fonction de critères fournis par l'utilisateur dans l'interface homme-machine.

La base de données est préférentiellement alimentée directement par les fabricants de dispositifs médicaux. Pour ce faire, les fabricants de dispositifs médicaux possèdent des moyens de connexion sécurisés à la base de données afin de déposer les plans de fabrication en trois dimensions.

Pour garantir la propriété des fichiers numériques du fabricant, la base de données peut être configurée pour chiffrer automatiquement les plans de fabrication reçus afin qu'ils ne puissent pas être récupérés par l'utilisateur mais uniquement utilisés par l'imprimante 3D (6) lorsque l'utilisateur les sélectionne.

En variante, les plans de fabrication correspondants aux dispositifs médicaux à imprimer peuvent être stockés uniquement sur un serveur du fabricant de dispositifs médicaux et accessibles uniquement lorsque l'utilisateur lance la commande d'impression. Ainsi, dans ce mode de réalisation, l'imprimante 3D (6) comporte des moyens pour se connecter à distance sur le serveur de chaque fabricant et avoir accès aux plans de fabrication du dispositif médical sélectionné par l'utilisateur.

De préférence, les plans de fabrication sont stockés dans la base de données chiffrées accessible par le module de gestion électronique de documents afin de pallier tout dysfonctionnement de réseau pour commander l'imprimante 3D (6).

Différents droits d'accès peuvent être mis en oeuvre pour restreindre l'accès aux informations contenues dans cette base de données. Par exemple, les fabricants peuvent avoir le droit de modifier les tarifs et les plans de chacun de leurs dispositifs médicaux. Pour autant, un fabricant de dispositif médical particulier n'a pas accès aux plans, ni même aux éléments de prévisualisation d'un dispositif médical d'un autre fabricant. En outre, un dispositif médical peut-être implanté dans le corps d'un patient par le biais d'un instrument d'implantation. Pour faciliter la réalisation ou la sélection d'instruments d'implantation en fonction du dispositif médical sélectionné par l'utilisateur, les fabricants d'instruments d'implantation peuvent également avoir accès à tout ou partie des informations de la base de données.

De même, les hôpitaux sont souvent gérés par des acheteurs ou des services financiers dont la préoccupation principale est de rationaliser les coûts de fonctionnement d'un hôpital. Ces services financiers peuvent également avoir accès à certaines informations techniques des dispositifs médicaux proposés par les fabricants afin de définir des stratégies d'achat de certains dispositifs médicaux en fonction du coût ou des relations commerciales établies avec un fabricant spécifique.

En complément de ces acteurs classiques de l'hôpital et de la fourniture d'instruments d'implantation ou de dispositif médical, un utilisateur de gestion de multiples installations (1) d'impression en trois dimensions peut également avoir accès à certaines informations contenues dans cette base de données afin pouvoir réaliser des statistiques sur l'utilisation d'un ou plusieurs dispositifs médicaux en fonction des recherches réalisées par l'utilisateur sur l'interface homme-machine.

Cette analyse d'informations permet d'améliorer l'expérience utilisateur dans la présentation des informations sur l'interface homme-machine afin de conseiller les dispositifs les plus souvent utilisés par les utilisateurs et, ainsi, de réduire le temps de sélection des dispositifs les plus couramment utilisés.

L'utilisateur de gestion peut également intervenir sur la base de données pour accéder à l'ensemble des profils des fabricants, des utilisateurs ou des services financiers afin de restreindre ou d'augmenter leur droit d'accès aux différentes informations contenues dans la base de données en fonction de contraintes spécifiques.

Ainsi, en plus du module de gestion électronique de documents, le serveur comporte préférentiellement un module de sécurisation permettant un contrôle d'accès sécurisé aux différents intervenants en fonction de leur droit d'accès. Ce module de sécurisation comprend également des moyens de chiffrement des plans de fabrication et des moyens de signature numérique permettant de tracer et légaliser une demande de production d'un utilisateur. Le serveur peut également comporter un module de statistique afin de faciliter l'analyse de l'utilisateur de gestion.

Ce serveur peut être intégré dans un système d'information global permettant de contrôler chaque étape du procédé de fabrication et de vérifier la conformité des pièces fabriquées par l'imprimante 3D (6) en utilisant les plans de fabrication stockés dans la base de données.

De ce qui précède, l'utilisateur final, tel qu'un chirurgien par exemple, pourra consulter librement, dans la base de données, les fichiers des dispositifs médicaux à imprimer, ainsi que leur documentation associée.

Ainsi, après avoir choisi le dispositif médical le mieux adapté au patient, l'utilisateur sélectionne la quantité, et éventuellement l'adresse de livraison en interne dans le lieu où se trouve le container (2), par exemple service orthopédique, etc.

Le cycle peut ensuite débuter dans le module de production (3) par une étape de préparation et de mélange de la poudre, éventuellement recyclée.

L'imprimante 3D (6) est ensuite préparée et lancée pour l'impression par frittage sélectif par laser de la poudre. Une fois l'impression terminée, le dispositif médical imprimé est déballé d'un bloc de poudre issu de l'imprimante 3D (6). La poudre est superflue est alors réintégrée dans la mélangeuse (7).

Le dispositif médical déballé est ensuite acheminé vers la station de dépoudrage (9) pour enlever tous les résidus de poudre, de préférence par sablage. Il peut également être nécessaire de faire subir une opération de tribofinition au dispositif médical pour obtenir un état de surface optimal.

Une fois dépoudré, le dispositif médical peut être prélavé et est ensuite envoyé vers la station de contrôle et de reprise (10).

Si le dispositif médical ne répond pas aux attentes, il est refusé, et par exemple jeté dans les moyens de collecte en vue de son recyclage.

S'il répond aux attentes, il est acheminé vers la salle blanche (4), en passant par le sas (11). Le dispositif médical est lavé et désinfecté dans des moyens de lavage et de désinfection (12), puis emballé. Le dispositif médical emballé est ensuite stérilisé dans un autoclave, éventuellement situé dans la salle blanche (4).

Le produit est ensuite sur-emballé et est stocké en attente de livraison. Le produit est ensuite livré, notamment dans la salle du centre hospitaliers où la pratique médicale doit être effectuée.

De ce qui précède, l'installation (1) selon l'invention permet d'éviter les aléas liés aux traitements et à la livraison des dispositifs médicaux puisque ceux-ci sont fabriqués directement sur place, à la demande. Les dispositifs médicaux sont emballés et identifiés selon les standards du lieu dans lequel le container (2) est installé, ce qui permet de simplifier le traçage et le suivi desdits dispositifs médicaux.

La base de données accessible par l'interface homme-machine permet à l'utilisateur d'interagir avec un seul interlocuteur tout en ayant accès à une pluralité de dispositif médicaux, de fabricant différents. Le coût et le temps de commande et de gestion desdits dispositifs médicaux sont diminués.

## Revendications

1. Installation (1) d'impression en trois dimensions d'un dispositif médical directement sur le lieu où le dispositif médical doit être utilisé, comprenant un container (2) comprenant intérieurement :
- un module de production (3) comprenant une imprimante 3D (6) ;
l'installation étant **caractérisée en ce que** le container comprend également
- une salle blanche (4), distincte du module de production (3), comprenant des moyens de lavage et de désinfection (12) du dispositif médical imprimé, et une machine d'emballage (13) du dispositif médical lavé et désinfecté.

2. Installation (1) selon la revendication 1, ***caractérisée* en ce que** l'imprimante 3D (6) est une machine de frittage sélectif de poudre par laser, et le module de production (3) comprend une station de déballage (8) d'un dispositif médical imprimé, et une station de dépoudrage (9) d'un dispositif médical déballé.

3. Installation (1) selon la revendication 2, ***caractérisée* en ce que** le module de production (3) comprend une mélangeuse (7) de poudre.

4. Installation (1) selon la revendication 2, ***caractérisée* en ce que** la station de dépoudrage (9) comprend un dispositif de dépoudrage par sablage.

5. Installation (1) selon l'une des revendications précédentes, ***caractérisée* en ce que** le module de production (3) comprend une station de contrôle et de reprise (10) du dispositif médical imprimé.

6. Installation (1) selon l'une des revendications précédentes, ***caractérisée* en ce que** la salle blanche (4) comprend un dispositif de stérilisation (15), de préférence de type autoclave.

7. Installation (1) selon l'une des revendications précédentes, ***caractérisée* en ce que** le module de production (3) communique avec la salle blanche (4) par l'intermédiaire d'un sas (11).

8. Installation (1) selon l'une des revendications précédentes, ***caractérisé* en ce que** l'imprimante 3D (6) est commandée par une interface homme-machine configurée pour permettre à un utilisateur de sélectionner un dispositif médical à imprimer accessible depuis une base de données.

9. Installation (1) selon l'une des revendications précédentes, ***caractérisé* en ce que** le container (2) comprend des moyens de collecte des dispositifs médicaux imprimés à jeter.

## Patentansprüche

1. Anlage (1) zum dreidimensionalen Drucken einer medizinischen Vorrichtung direkt an dem Ort, an dem die medizinische Vorrichtung verwendet werden soll, umfassend einen Container (2), der im Inneren Folgendes umfasst:
- ein Produktionsmodul (3), das einen 3D-Drucker (6) umfasst;
wobei die Anlage ***dadurch gekennzeichnet* ist, dass** der Container auch umfasst:
- einen Reinraum (4), der vom Produktionsmodul (3) getrennt ist und Mittel zum Waschen und Desinfizieren (12) der gedruckten medizinischen Vorrichtung umfasst, und eine Verpackungsmaschine (13) für die gewaschene und desinfizierte medizinische Vorrichtung.

2. Anlage (1) nach Anspruch 1, ***dadurch gekennzeichnet,* dass** der 3D-Drucker (6) eine Maschine zum selektiven Lasersintern von Pulver ist und das Produktionsmodul (3) eine Auspackstation (8) für eine gedruckte medizinische Vorrichtung und eine Entpuderungsstation (9) für eine ausgepackte medizinische Vorrichtung umfasst.

3. Anlage (1) nach Anspruch 2, ***dadurch gekennzeichnet,* dass** das Produktionsmodul (3) einen Pulvermischer (7) umfasst.

4. Anlage (1) nach Anspruch 2, ***dadurch gekennzeichnet,* dass** die Entpuderungsstation (9) eine Sandstrahlentpuppungsvorrichtung umfasst.

5. Anlage (1) nach einem der vorhergehenden Ansprüche, ***dadurch gekennzeichnet,* dass** das Produktionsmodul (3) eine Kontroll- und Übernahmestation (10) für die gedruckte medizinische Vorrichtung umfasst.

6. Anlage (1) nach einem der vorhergehenden Ansprüche, ***dadurch gekennzeichnet,* dass** der Reinraum (4) eine Sterilisationsvorrichtung (15), vorzugsweise vom Typ Autoklav, umfasst.

7. Anlage (1) nach einem der vorhergehenden Ansprüche, ***dadurch gekennzeichnet,* dass** das Produktionsmodul (3) über eine Schleuse (11) mit dem Reinraum (4) in Verbindung steht.

8. Anlage (1) nach einem der vorhergehenden Ansprüche, ***dadurch gekennzeichnet,* dass** der 3D-Drucker (6) von einer Mensch-Maschine-Schnittstelle gesteuert wird, die so konfiguriert ist, dass ein Benutzer eine zu druckende medizinische Vorrichtung auswählen kann, auf die von einer Datenbank aus zugegriffen werden kann.

9. Anlage (1) nach einem der vorhergehenden Ansprüche, ***dadurch gekennzeichnet,* dass** der Behälter (2) Mittel zum Sammeln von wegzuwerfenden gedruckten medizinischen Geräten umfasst.

## Claims

1. Three-dimensional printing installation (1) of a medical device directly at a location where the medical device is to be used, comprising a container (2) comprising inside it:
- a production module (3) comprising a 3D printer (6);
the installation being **characterised in that** the container also comprises:
- a clean room (4), separate from the production module (3), comprising means (12) for washing and disinfecting the printed medical device, and a machine (13) for packaging the washed and disinfected medical device.

2. Installation (1) according to claim 1, ***characterised* in that** the 3D printer (6) is a powder selective laser sintering machine, and the production module (3) comprises a station (8) for unpackaging a printed medical device, and a station (9) for de-powdering an unpackaged medical device.

3. Installation (1) according to claim 2, ***characterised* in that** the production module (3) comprises a powder mixer (7).

4. Installation (1) according to claim 2, ***characterised* in that** the de-powdering station (9) comprises a sandblasting de-powdering device.

5. Installation (1) according to one of the preceding claims, ***characterised* in that** the production module (3) comprises a control and rework station (10) for the printed medical device.

6. Installation (1) according to one of the preceding claims, ***characterised* in that** the clean room (4) comprises a sterilisation device (15), preferably of the autoclave type.

7. Installation (1) according to one of the preceding claims, ***characterised* in that** the production module (3) communicates with the clean room (4) by way of an airlock (11).

8. Installation (1) according to one of the preceding claims, ***characterised* in that** the 3D printer (6) is controlled by a man-machine interface configured to make it possible for a user to select a medical device to be printed, accessible from a database.

9. Installation (1) according to one of the preceding claims, ***characterised* in that** the container (2) comprises means for collecting printed medical devices to be discarded.
